# EUROPÄISCHE PATENTSCHRIFT

(11) **EP 1 997 794 B1**
(45) Veröffentlichungstag und Bekanntmachung des Hinweises auf die Patenterteilung: **30.03.2011**
(21) Anmeldenummer: 08154500.6
(22) Anmeldetag: 15.04.2008
(51) Int. Cl.: C07C 29/70

(54) **Verfahren zur Herstellung von Alkalimetallalkoholaten**
Method for manufacturing alkali metal alcoholates
Procédé de fabrication d'alcoolates de métaux alcalins

(30) Priorität: 01.06.2007 DE 102007025904
(43) Veröffentlichungstag der Anmeldung: 03.12.2008
(73) Patentinhaber: Evonik Degussa GmbH, 45128 Essen (DE)
(72) Erfinder: Ruwwe, Johannes, 53859, Niederkassel (DE); Krüger, Kai-Martin, 45145, Essen (DE); Knippenberg, Udo, 45772, Marl (DE); Brehme, Volker, 48301, Nottuln-Appelhülsen (DE); Neumann, Manfred, 45770, Marl (DE)

(56) Entgegenhaltungen:
- EP-A- 1 242 345

## Beschreibung

Die vorliegende Erfindung beschreibt ein Verfahren zur Herstellung von Alkalimetallalkoholaten aus Alkalimetallhydroxiden mittels Reaktivdestillation.

Alkalimetallalkoholate werden als starke Basen in der Synthese zahlreicher Chemikalien, z.B. bei der Herstellung von Pharma- oder Agrowirkstoffen eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion entstehende Reaktionswasser mit dem Destillat entfernt wird.

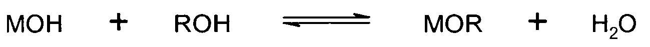

Ein solches Verfahrensprinzip ist beispielsweise in der US 2,877,274 beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der EP 1 242 345 erneut beschrieben.

Ähnliche Verfahren, bei denen jedoch zusätzlich noch ein Schleppmittel, wie beispielsweise Benzol, eingesetzt wird, sind in der GB 377,631 und der US 1,910,331 beschrieben. Das Schleppmittel dient hierbei, um eine Trennung von Wasser und einem wasserlöslichen Alkohol zu ermöglichen. Bei beiden Patentschriften wird das Kondensat einer Phasentrennung unterzogen, um das Reaktionswasser abzutrennen.

So beschreibt auch die DE 96 89 03 ein Verfahren zur kontinuierlichen Herstellung von Alkalialkoholaten, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577, wobei die Wasserabtrennung im Kondensat mit Hilfe einer Membran erfolgt.

Sämtliche Verfahren weisen in der Praxis den Nachteil auf, dass an der Zulaufstelle der Alkalimetallhydroxid-Lösung - also an der Stelle des Kolonnenprofils, an der die Konzentration des eingesetzten Alkalimetallhydroxids am größten ist, die Gefahr besteht, dass eine Feststoffausfällung auftritt und der Prozess unterbrochen werden muss.

Es war deshalb die Aufgabe der vorliegenden Erfindung ein Verfahren zur Herstellung einer alkoholischen Lösung eines Alkalimetallalkoholats bereitzustellen, das einen stabilen, kontinuierlichen Herstellungsprozess mit einer hohen Verfahrenssicherheit bietet, bei dem handelsübliche wässrige Alkalimetallhydroxid-Lösungen eingesetzt werden können.

Überraschenderweise konnte ein Verfahren zur Herstellung einer alkoholischen Lösung eines Alkalimetallalkoholats aus Alkalimetallhydroxid und Alkohol in einer Reaktionskolonne gefunden werden, wobei der Alkohol und das Alkalimetallhydroxid im Gegenstrom geführt werden, das sich dadurch auszeichnet, dass ein Rücklaufverhältnis von mindestens 0,05 an der Reaktionskolonne eingestellt wird. Dies bedeutet bei einer vollständigen Kondensation, dass mindestens 5 Gew.- % dieses Kondensats der Reaktionskolonne in Form eines Rücklaufes am Kopf der Reaktionskolonne zurückgeführt werden. Durch das Einstellen eines Rücklaufverhältnisses an der Reaktionskolonne kann überraschenderweise vermieden werden, dass es unterhalb der Zulaufstelle der Alkalimetallhydroxid-Lösung zu einer Feststoffausfällung kommt. Gegenüber den Verfahren gemäß dem Stand der Technik hat das erfindungsgemäße Verfahren den Vorteil, dass die Löslichkeitsgrenze des Alkalimetallhydroxids an der Zulaufstelle der Alkalimetallhydroxid-Lösung - an der die Konzentration des eingesetzten Alkalimetallhydroxids am größten ist - nicht überschritten wird. Das einzustellende Rücklaufverhältnis in dem erfindungsgemäßen Verfahren richtet sich nach der Art des herzustellenden Alkalimetallalkoholats, bzw. nach der Löslichkeit des entsprechenden Alkalimetallhydroxids in dem sich einstellenden Gemisch aus Wasser und dem eingesetzten Alkohol. Insbesondere war dies überraschend, da die Verfahren gemäß dem Stand der Technik ohne Rücklauf in der Reaktionskolonne durchgeführt werden oder dem Kondensat mittels Phasentrennung oder Membrantechniken das Wasser entzogen wird, bevor der nun wasserfreie Rücklauf der Reaktionskolonne zurückgeführt wird. Die Einstellung eines Rücklaufverhältnisses sorgt dafür, dass im oberen Teil der Reaktionskolonne immer eine ausreichend hohe Wasserkonzentration vorliegt, so dass eine Feststoffausfällung wirkungsvoll verhindert wird. Somit ist ein Verfahren möglich, das unempfindlicher gegenüber Störungen oder Schwankungen, wie beispielsweise Konzentrationsschwankungen in den Zuläufen, ist. Im Gegensatz zu den Verfahren gemäß dem Stand der Technik erfolgt in dem erfindungsgemäßen Verfahren keine Abtrennung des Wassers aus dem Kondensat bevor der Rücklauf in die Reaktionskolonne zurückgeführt wird.

Gegenstand der Erfindung ist somit ein Verfahren zur Herstellung einer alkoholischen Lösung eines Alkalimetallalkoholats aus Alkalimetallhydroxid und Alkohol in einer Reaktionskolonne, wobei der Alkohol und das Alkalimetallhydroxid im Gegenstrom geführt werden, das sich dadurch auszeichnet, dass ein Rücklaufverhältnis von mindestens 0,05 an der Reaktionskolonne eingestellt wird.

Das Rücklaufverhältnis richtet sich in dem erfindungsgemäßen Verfahrens nach der Art des herzustellenden Alkalimetallalkoholats, bzw. nach der Löslichkeit des eingesetzten Alkalimetallhydroxids in dem sich einstellenden Gemisch aus Wasser und dem eingesetzten Alkohol. Als Brüden der Reaktionskolonne erhält man den Alkohol mit einer geringen Menge an Wasser, wobei in diesen erfindungsgemäßen Verfahren mindestens 5 Gew.-% dieser Brüden kondensiert und dieses Kondensat in Form eines Rücklaufs am Kopf der Reaktionskolonne zurückgefahren werden, dies entspricht einem Rücklaufverhältnis von mindestens 0,05 an der Reaktionskolonne. Unter einem Rücklaufverhältnis wird im Sinne dieser Erfindung das Verhältnis des Massenstroms (kg/h), der der Kolonne in flüssiger Form zurückgeführt wird (Rücklauf) und des Massenstroms (kg/h), der in flüssiger Form (Destillat) oder gasförmiger Form (Brüden) von der Reaktionskolonne abgezogen wird. Bevorzugt wird an der Reaktionskolonne ein Rücklaufverhältnis von 0,11 bis 0,34, besonderes bevorzugt von 0,14 bis 0,27 und ganz besonders bevorzugt ein Rücklaufverhältnis von 0,17 bis 0,24 eingestellt. Somit werden bevorzugt von 10 bis 25 Gew.-%, besonders bevorzugt von 12 bis 21 Gew.-% und ganz besonders bevorzugt von 15 bis 19 Gew.-% des Kopfproduktes in Form eines Rücklaufs am Kopf der Reaktionskolonne in diese zurückgeführt. In dem erfindungsgemäßen Verfahren wird das Wasser im Kondensat und/oder im Rücklauf der Reaktionskolonne vor der Rückführung als Rücklauf in die Reaktionskolonne vorzugsweise nicht abgetrennt. Die Einstellung eines Rücklaufverhältnisses in der Reaktionskolonne sorgt dafür, dass im oberen Teil der Gegenstromdestillationskolonne immer eine ausreichend hohe Wasserkonzentration im Reaktionsgemisch vorliegt, so dass eine Feststoffausfällung wirkungsvoll verhindert werden kann.

Als mögliche Alkalimetallhydroxide können in dem erfindungsgemäßen Verfahren Natriumhydroxid, Kaliumhydroxid oder Lithiumhydroxid eingesetzt werden, bevorzugt werden Natriumhydroxid und Kaliumhydroxid verwendet. Die Alkalimetallhydroxide können in dem erfindungsgemäßen Verfahren in Form einer wässrigen, alkoholischen oder einer wässrigen Lösung, die den eingesetzten Alkohol als weiteres Lösemittel aufweist, eingesetzt werden.

Die Alkalimetallhydroxide werden in dem erfindungsgemäßen Verfahren vorzugsweise als wässrige Lösung eingesetzt, bevorzugt wird eine wässrige Lösung mit einem Gehalt (acidimetrisch) an Alkalimetallhydroxid von 15 bis 55 %, besonders bevorzugt von 30 bis 53 % und besonders bevorzugt von 45 bis 52 %, eingesetzt.

In dem erfindungsgemäßen Verfahren können als Alkohole aliphatische Alkohole mit 1 bis 5 Kohlenstoffatomen eingesetzt werden, wie beispielsweise Methanol, Ethanol, n- oder iso-Propanol, n-, sec-, iso- oder tert-Butanol, oder Isomere des Pentanols, wie beispielsweise n-Pentanol. Bevorzugt werden in dem erfindungsgemäßen Verfahren jedoch Methanol oder Ethanol eingesetzt, besonders bevorzugt wird Methanol eingesetzt.

Der in dem erfindungsgemäßen Verfahren eingesetzte Alkohol kann sowohl als Lösemittel als auch als Edukt dienen. Weiterhin kann der eingesetzte Alkohol dazu dienen, Wasser aus der Flüssigkeit herauszustrippen, so dass dieses über das Destillat aus dem Reaktionsraum entfernt werden kann. Daher wird in dem erfindungsgemäßen Verfahren vorzugsweise die 15 bis 45 fache Menge, bevorzugt die 25 bis 35 fache Menge, an Alkohol bezogen auf das Gewicht an Wasser im Zulaufstrom des Alkalimetallhydroxids eingesetzt.

Es ist vorteilhaft die Reaktionskolonne bei einem Druck von 0,5 bis 2 bar zu betreiben, bevorzugt wird die Reaktionskolonne bei Umgebungsdruck betrieben.

Die Alkalimetallhydroxid-Lösung wird in dem erfindungsgemäßen Verfahren vorzugsweise auf den obersten Boden zugegeben bzw. am Kopf der Reaktionskolonne zugeführt. Von Vorteil für das erfindungsgemäße Verfahren ist es, die Alkalimetallhydroxid-Lösung erwärmt, bevorzugt mit einer Temperatur knapp unterhalb des Siedepunktes dieser Lösung, der Reaktionskolonne zuzuführen. Bei der Verwendung einer wässrigen Natriumhydroxid-Lösung wird diese vor der Zufuhr in die Reaktionskolonne vorzugsweise auf eine Temperatur von 50 bis 70 °C erhitzt, beispielsweise mittels eines Wärmetauschers. Die Zulauftemperatur der Alkalimetallhydroxid-Lösung sollte die Temperatur des Bodens an der Zulaufstelle der Alkalimetallhydroxid-Lösung aufweisen.

In dem erfindungsgemäßen Verfahren wird vorzugsweise das Kopfprodukt, also Brüden oder Destillat, welches nicht als Rücklauf der Reaktionskolonne zurückgeführt wird, in einem weiteren Verfahrensschritt in Wasser und Alkohol aufgetrennt - bevorzugt kann dies in einer Rektifikationskolonne erfolgen, wobei der durch die Trennstufe gewonnene Alkohol der Reaktionskolonne als zurückgeführter Alkohol zugeführt werden kann.

Der Reaktionskolonne in dem erfindungsgemäßen Verfahren kann somit sowohl zurückgeführter Alkohol aus der Rektifikationskolonne als auch Frischalkohol zugeführt werden. Bevorzugt wird der Reaktionskolonne sowohl zurückgeführter Alkohol als auch Frischalkohol zugeführt. Wahlweise kann der Frischalkohol der Reaktionskolonne in einer separaten Zulaufstelle oder zusammen mit dem zurückgeführten Alkohol der Reaktionskolonne zugeführt werden.

Der zurückgeführte Alkohol aus der Rektifikationskolonne wird vorzugsweise gasförmig mindestens zwanzig Böden unterhalb der Zulaufstelle der Alkalimetallhydroxid-Lösung der Reaktionskolonne zugeführt. Bevorzugt wird der zurückgeführte Alkohol 1 bis 10 Böden, besonders bevorzugt 1 bis 6 Böden oberhalb des Sumpfes oder direkt in den Sumpf der Reaktionskolonne zudosiert. Ganz besonders bevorzugt wird der zurückgeführte Alkohol in den Sumpf der Reaktionskolonne zudosiert.

Der Frischalkohol kann in dem erfindungsgemäßen Verfahren sowohl als Dampf als auch flüssig eingesetzt werden. Bevorzugt wird der Frischalkohol in den Sumpf, in den Verdampfer oder auf einen Boden oder auf mehrere Böden, die 1 bis 10, besonders bevorzugt 1 bis 6 Böden oberhalb des Sumpfes sich befinden, zugeführt. Insbesondere erfolgt die Zufuhr des Frischalkohols in dem erfindungsgemäßen Verfahren in den Sumpf oder in den Verdampfer der Reaktionskolonne - vorzugsweise gasförmig.

In einer bevorzugten Ausführungsform des erfindungsgemäßen Verfahrens wird Frischalkohol in den oberen Teil der Rektifikationskolonne - insbesondere zusammen mit dem Rücklauf der Rektifikationskolonne zugegeben. Diese Ausführungsform des erfindungsgemäßen Verfahrens eignet sich insbesondere, um überschüssiges Wasser aus dem Methanol zu entfernen, bevor der Alkohol in die Reaktionskolonne geführt wird. Besonders bevorzugt erfolgt die Zugabe des Frischalkohols hierbei in den Kondensator oder in den Kondensatbehälter an der Rektifikationskolonne, ganz besonders bevorzugt erfolgt die Zugabe des Frischalkohols in den Kondensator an der Rektifikationskolonne.

In einer besonderen Ausführungsform des erfindungsgemäßen Verfahrens wird der Frischalkohol in den Rücklauf der Reaktionskolonne gegeben, dies kann hierbei in den Kondensator als auch in den Kondensatbehälter an der Reaktionskolonne erfolgen.

Der in die Reaktionskolonne zudosierte Alkohol - sowohl der zurückgeführte Alkohol als auch der Frischalkohol - weisen im Durchschnitt vorzugsweise einen Wassergehalt von maximal 1000 ppm (m/m), bevorzugt maximal 500 ppm (m/m) auf. Besonders vorteilhaft ist es für das erfindungsgemäße Verfahren, wenn der in die Reaktionskolonne zudosierte Alkohol einen durchschnittlichen Wassergehalt von 200 bis 450 ppm (m/m), bevorzugt von 300 bis 400 ppm (m/m) aufweist.

Das erfindungsgemäße Verfahren kann sowohl kontinuierlich als auch halbkontinuierlich betrieben werden. Beim halbkontinuierlichen Betrieb wird der Alkohol, vorzugsweise Methanol, im Verdampfer der Reaktionskolonne vorgelegt, während des Betriebs wird der Alkohol dem Kolonnensumpf in flüssiger Form, oder gasförmig am untersten oder einem der unteren Böden der Kolonne und eine Alkalimetallhydroxid-Lösung am Kopf der Reaktionskolonne zudosiert.

Bevorzugt wird das erfindungsgemäße Verfahren jedoch kontinuierlich betrieben. Hierbei wird bevorzugt am Kopf der Reaktionskolonne Alkalimetallhydroxid-Lösung zudosiert, während dem Sumpf der Reaktionskolonne gasförmiger Alkohol zudosiert wird.

Die Reaktionskolonne des erfindungsgemäßen Verfahrens weist bevorzugt Kolonnenböden von Typ Glockenboden, ausgewählt aus Glockenböden, Tunnelböden, Thormann-Böden oder Kreuzschlitzglockenböden, als Einbauten auf. In einer weiteren Ausführungsform des erfindungsgemäßen Verfahrens kann die Reaktionskolonne auch Sieb- oder Ventilböden, strukturierte oder unstrukturierte Packungen oder Füllkörper aufweisen.

Der Produktstrom wird in dem erfindungsgemäßen Verfahren vorteilhafterweise am Kolonnensumpf bzw. Verdampfer entnommen und besteht aus einer alkoholischen Lösung des gewünschten Alkalimetallalkoholats, beispielsweise als 30%ige Lösung. Dieser Produktstrom kann gegebenenfalls geringe Mengen an Wasser und / oder nicht abreagiertem Alkalimetallhydroxid aufweisen.

Das nicht in die Reaktionskolonne zurückgeführte Destillat wird in dem erfindungsgemäßen Verfahren vorzugsweise in einem separaten Schritt aufgetrennt und somit kann der vom Wasser befreite Alkohol in die Reaktionskolonne zurückgeführt werden. Bevorzugte Verfahren für diese Aufarbeitung des Alkohols sind Destillation, Trocknung mittels Membranverfahren oder Trocknung mittels eines Molekularsiebs, wie sie dem Fachmann bekannt sind. Bevorzugt wird diese Aufarbeitung des Alkohols in dem erfindungsgemäßen Verfahrens mittels Destillation durchgeführt.

In einer besonders bevorzugten Ausführungsform des erfindungsgemäßen Verfahrens wird das am Kopf der Reaktionskolonne entnommene und nicht als Rücklauf in die Reaktionskolonne zurückgeführte Kopfprodukt in einer Rektifikationskolonne bei höherem Druck als in der Reaktionskolonne aufgearbeitet. Von Vorteil für das erfindungsgemäße Verfahren ist es dabei, diesen Teil des Kopfproduktes der Reaktionskolonne über einen Brüdenverdichter in die Rektifikationskolonne zu führen. Über den Sumpfaustrag der Rektifikationskolonne wird das Wasser aus dem System ausgeschleust. Das am Kolonnenkopf der Rektifikationskolonne erhaltene Destillat kann der Reaktionskolonne wiederum zurückgeführt werden.

Ebenso ist es möglich, die Reaktion bei höherem Druck als die Rektifikation durchzuführen und das Kopfprodukt der Rektifikationskolonne mittels eines Brüdenverdichters in die Reaktionskolonne zurückzuführen.

Die Rektifikation des Gemischs aus Alkohol und Wasser kann in dem erfindungsgemäßen Verfahren gemäß bekannten Verfahrensschritten erfolgen, wie sie beispielsweise in "Distillation: Principles and Practices" (Johann G. Stichlmair, James R. Fair; Wiley-VCH) beschrieben sind. Bei der Verwendung von Methanol als Alkohol in dem erfindungsgemäßen Verfahren wird das Destillat der Reaktionskolonne bevorzugt in einer Rektifikationskolonne aufgearbeitet. Als Einbauten eignen sich hierbei alle bekannten Einbauten, sowohl Böden als auch strukturierte Packungen und Füllkörperschüttungen. Das Methanol fällt hierbei als leichtsiedendes Destillat am Kopf der Rektifikationskolonne an und das Wasser kann am Sumpf der Rektifikationskolonne entnommen werden. Das Rücklaufverhältnis ist hierbei vorzugsweise größer als 0,5 und beträgt bevorzugt von 0,8 bis 1,5. Der Druck in der Rektifikationskolonne kann sowohl niedriger als auch höher als in der Reaktionskolonne gewählt werden. Die Temperatur stellt sich dementsprechend ein. Der Methanol/Wasser-Zulauf wird in die untere Hälfte der Rektifikationskolonne gegeben, bevorzugt zwischen dem 2. bis 15. Boden oberhalb des Verdampfers. In dieser bevorzugten Ausführungsform des erfindungsgemäßen Verfahrens wird Frischalkohol in den oberen Teil der Rektifikationskolonne - insbesondere zusammen mit dem Rücklauf der Rektifikationskolonne zugegeben, um überschüssiges Wasser aus dem Methanol zu entfernen, bevor der Alkohol in die Reaktionskolonne zurückgeführt wird. Besonders bevorzugt erfolgt die Zugabe des Frischalkohols hierbei in den Kondensator oder in den Kondensatbehälter an der Rektifikationskolonne, ganz besonders bevorzugt erfolgt die Zugabe des Frischalkohols in den Kondensator an der Rektifikationskolonne.

Bei der Verwendung von Ethanol, n-, oder iso-Propanol, n-, sec-, iso- oder tert-Butanol, oder bei den Isomeren des Pentanols werden ebenfalls bevorzugt destillative ggf. mehrstufige Verfahren für die Aufarbeitung des Alkohols in dem erfindungsgemäßen Verfahren gewählt, bei denen mittels der dem Fachmann bekannten Techniken die Azeotrope bzw. Hetero-Azeotrope aufgetrennt werden können. Als Hilfsmittel können Azeotropbildner gemäß des Standes der Technik eingesetzt werden, die in der Lage sind, mit dem Alkohol Heteroazeotrope auszubilden. Es können jedoch auch Adsorbentien, wie beispielsweise Zeolithe, hierfür eingesetzt werden.

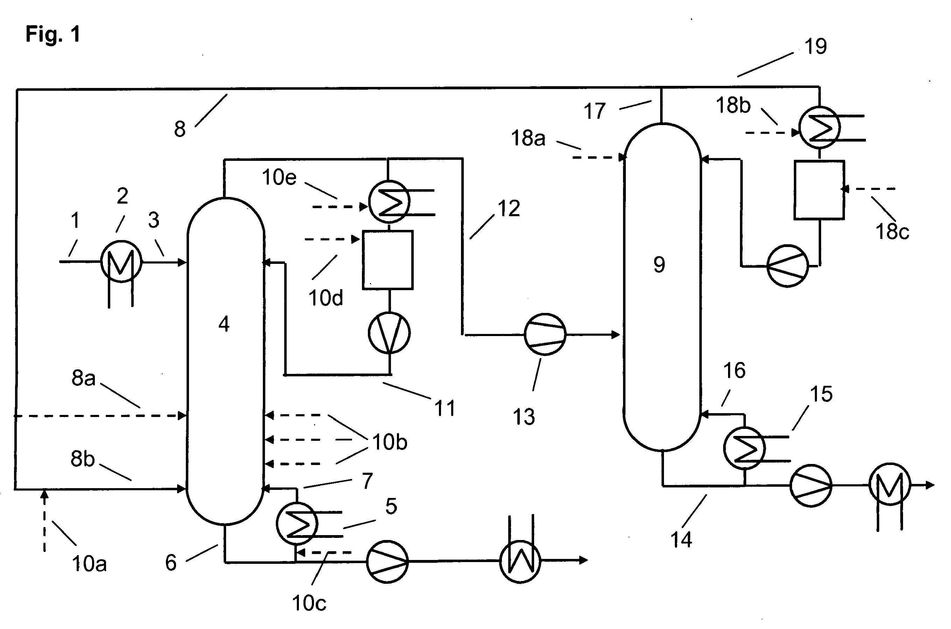

Figur 1 zeigt eine Anlage mit einem geringeren Druck in der Reaktionskolonne 4 als in der Rektifiktionskolonne 9. Hierbei wird die Alkalimetallhydroxid-Lösung über die Rohrleitung 1 in einen Wärmetauscher 2 geführt, in der sie bis auf die Temperatur der Zulaufstelle aufgeheizt und über die Rohrleitung 3 am Kopf der Kolonne 4 zugegeben wird. Die Alkalimetallalkoholatlösung wird am Sumpf der Kolonne über die Rohrleitung 6 entnommen. Am Sumpf der Kolonne befindet sich ein Verdampfer 5, mit dem die Konzentration der Alkalimetallalkoholatlösung auf den gewünschten Wert eingestellt wird. Die Brüden aus dem Verdampfer 5 werden über die Rohrleitung 7 am unteren Ende der Reaktionskolonne 4 zugegeben. Die Einspeisung des zurückgeführten Alkohols 8 aus der Rektifikationskolonne kann an mehreren Stellen der Reaktionskolonne 4 erfolgen: 8a und 8b. Neben der Einspeisung des zurückgeführten Alkohols kann auch Frischalkohol über die Zuleitungen 10a bis 10e erfolgen. Am Kopf der Kolonne wird ein Teil der Brüden kondensiert und als Rücklauf 11 in die Reaktionskolonne 4 wieder zurückgeführt. Der andere Teil der Brüden wird über eine Rohrleitung 12 und einem Brüdenverdichter 13 in die Rektifikationskolonne 9 zugefahren. Am Sumpf dieser Rektifikationskolonne 9 wird Wasser über die Rohrleitung 14 entnommen. Die Kolonne wird über den Verdampfer 15 beheizt, der den Brüdenstrom über die Rohrleitung 16 am unteren Ende der Rektifikationskolonne 9 zuführt. Am Kopf der Rektifikationskolonne 9 kann über die Rohrleitung 18a Frischalkohol auf den Kopf der Rektifikationskolonne aufgegeben werden. Am Kopf der Rektifikationskolonne wird ein Teil der Brüden kondensiert und als Rücklauf 19 in die Rektifikationskolonne 9 wieder zurückgeführt. Der andere Teil der Brüden wird über eine Rohrleitung 8 in die Reaktionskolonne 4 überführt. Die Zugabe des Frischalkohols kann auch in den Rücklauf der Rektifikationskolonne an den Zulaufstellen 18b und 18c erfolgen.

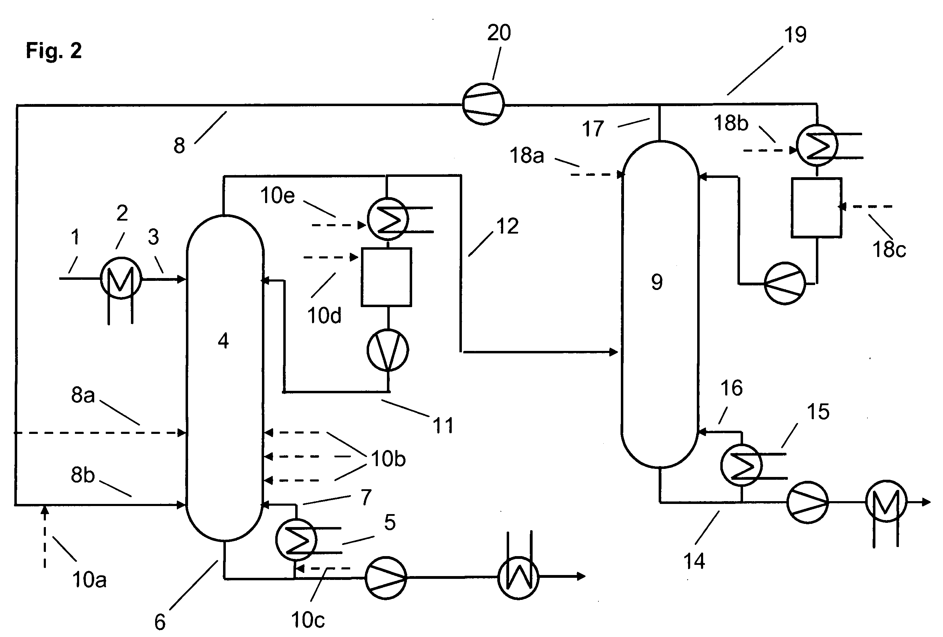

Figur 2 zeigt eine Anlage mit einem höheren Druck in der Reaktionskolonne 4 als in der Rektifiktionskolonne 9. Diese Anlage unterscheidet sich von der Anlage in Figur 1 dadurch, dass der Brüdenstrom 8 über einen Brüdenverdichter 20 in die Reaktionskolonne 4 geführt wird, Der Brüdenverdichter 13 entfällt.

Die nachfolgenden Beispiele sollen das erfindungsgemäße Verfahren zur Herstellung von Alkalimetallalkoholaten näher erläutern, ohne dass die Erfindung auf diese Ausführungsform beschränkt sein soll.

### Beispiele

### 1. Kontinuierliches Verfahren

### Beispiel 1.1 (erfindungsgemäß):

Am Kopf einer 9 Meter hohen mit 40 Glockenböden ausgerüsteten Reaktionskolonne und einem Innendurchmesser von 80 mm, werden 0,53 kg/h einer 50%igen wässrigen Natronlauge auf etwa 60°C erhitzt und dem obersten Boden zugeführt. Die Reaktionskolonne ist mit einer elektrischen Kompensationsheizung ausgerüstet, die jeweils 10 Böden umfasst, um Wärmeverluste an die Umgebung zu minimieren. Die Temperatur der Kompensationsheizung wird jeweils auf die Innentemperatur des mittleren Bodens des jeweiligen Abschnittes geregelt. 12 Gew.-% des Kondensats der Reaktionskolonne werden in Form eines Rücklaufs der Reaktionskolonne zurückgeführt, d.h. es wird ein Rücklaufverhältnis von 0,14 eingestellt, um den Anteil an gelöstem Feststoff in der Flüssigphase zu reduzieren. Ferner wird sie bei Umgebungsdruck betrieben. Zwischen dem Verdampfer und dem untersten Boden dieser Reaktionskolonne werden 8,88 kg/h dampfförmiges Methanol mit einem Wassergehalt von 400 ppm (m/m) und einer Temperatur von etwa 70 °C in die Reaktionskolonne geleitet, der im Gegenstrom zur flüssigen Phase in der Kolonne aufsteigt.

Dem Verdampfer der Reaktionskolonne wird ein Produktstrom von 1,16 kg/h entnommen, welcher aus einer 30%igen Natriummethylat-Lösung in Methanol besteht und dessen Konzentration von Wasser und nicht abreagiertem Natriumhydroxid - berechnet als Natriumhydroxid - in Summe bei 0,18 Gew.-% liegt. Die Temperatur am Kolonnenkopf liegt bei 75°C. Dort fällt eine Kondensatmenge von 8,25 kg/h an, die aus reinem Methanol mit einem 5%igen Wasseranteil besteht. Etwa 1 kg/h des Kondensats wird als Rücklauf in die Kolonne zurückgeführt.

Die Gesamtalkalität, d.h. die Konzentration an Natriumhydroxid und Natriummethylat in der Flüssigphase wird durch diese Maßnahme auf unter 20 Gew.-% reduziert, d.h. die Flüssigphase wird verdünnt und die Konzentrationen von Natriumhydroxid und Natriummethylat liegen deutlich unterhalb der jeweiligen Löslichkeitsgrenze. Dies gilt insbesondere für das Natriumhydroxid, da durch das zurückgeführte Wasser der Wasseranteil in der Flüssigphase deutlich erhöht wird. Das Natriumhydroxid verbleibt somit in Lösung. Es kann daher kein Feststoffausfall in der Reaktionskolonne beobachtet werden.

### Beispiel 1.2 (nicht erfindungsgemäß):

Am Kopf einer 9 Meter hohen mit 40 Glockenböden ausgerüsteten Reaktionskolonne und einem Innendurchmesser von 80 mm, werden 0,55 kg/h einer 50%igen wässrigen Natronlauge auf etwa 60°C erhitzt und dem obersten Boden zugeführt. Die Reaktionskolonne ist mit einer elektrischen Kompensationsheizung ausgerüstet, die jeweils 10 Böden umfasst, um Wärmeverluste an die Umgebung zu minimieren. Die Temperatur der Kompensationsheizung wird jeweils auf die Innentemperatur des mittleren Bodens des jeweiligen Abschnittes geregelt. Die Kolonne wird ohne Rücklauf gefahren, ferner wird sie bei Umgebungsdruck betrieben. Zwischen dem Verdampfer und dem untersten Boden dieser Reaktionskolonne werden 8,74 kg/h dampfförmiges Methanol mit einem Wassergehalt von 400 ppm (m/m) und einer Temperatur von etwa 70°C in die Reaktionskolonne geleitet, der im Gegenstrom zur flüssigen Phase in der Kolonne aufsteigt.

Dem Verdampfer der Reaktionskolonne wird ein Produktstrom von 1,27 kg/h entnommen, welcher aus einer 30 %igen Natriummethylat-Lösung in Methanol besteht und dessen Konzentration von Wasser und nicht reagiertem Natriumhydroxid - berechnet als Natriumhydroxid - in Summe bei 0,15 Gew.-% liegt. Die Temperatur am Kolonnenkopf liegt bei 75°C. Dort fällt eine Kondensatmenge von 8,01 kg/h an, die aus reinem Methanol mit einem 5%igen Wasseranteil besteht.

Die Gesamtalkalität, d. h. die Konzentration an Natriumhydroxid und Natriummethylat in der Flüssigphase liegt deutlich über 30 Gew.-%. Die Natriumhydroxid-Konzentration im oberen Teil der Kolonne ist deutlich höher und die Wasserkonzentration deutlich geringer als im Beispiel 1.1. Auf den oberen Böden der Kolonne wird Feststoffausfall beobachtet.

### 2. Halbkontinuierliches Verfahren

Eine Labor-Destillationsapparatur wird folgendermaßen aufgebaut: Ein Mehrhalskolben wird mit zwei Kolonnenschüssen, die über ein Zwischenstück zum Einbau einer Temperaturmessung verbunden sind, sowie mit Rührung, Thermometer und einer Sumpfdosierung ausgestattet. Als Kolonnenschüsse können wahlweise Boden- oder Packungskolonnen verwendet werden, es ist ebenfalls möglich oberhalb und unterhalb der Temperaturmessstelle unterschiedliche Kolonnenarten einzusetzen. Die Kolonne ist mit einem Rücklaufteiler ausgestattet. Zur Überprüfung der Feststoffbildung wird bevorzugt eine Glockenbodenkolonne aus Glas eingesetzt. Der Kopf der Destillationskolonne ist mit Kühler, Rücklaufteiler und einer Kopfdosierung ausgestattet. Die zulaufenden Ströme im Sumpf oder am Kopf können wahlweise beheizt werden.

### Beispiel 2.1 (erfindungsgemäß):

Als Reaktionskolonne wird folgender Aufbau gewählt: Unterhalb der Temperaturmessstelle wird ein Kolonnenschuss mit einer Stahlwollepackung (20 cm) und oberhalb eine Glockenbodenkolonne mit 9 Böden eingesetzt. Beide Kolonnen haben einen Durchmesser von 29 mm. Die Apparatur wird mit 720 g Methanol beschickt, welches zum Sieden erhitzt wird. Der Rücklaufteiler wird auf ein Rücklaufverhältnis von 0,16 eingestellt, so dass ca. 300 g Destillat pro h erhalten werden. Am Kopf der Reaktionskolonne wird mit einer Dosierung von 13 g/h eine 50%ige wässrige Natronlauge zudosiert. Über die Sumpfdosierung werden 290 g/h Methanol zudosiert. Es ist keine Feststoffausfällung zu beobachten.

Nach 6 Stunden wird die Dosierung der Natronlauge am Kopf beendet. Für weitere 30 Minuten wird Methanol am Sumpf der Kolonne zudosiert und mit gleichbleibender Kondensatabnahme weiterdestilliert. Man erhält nach 6,5 Stunden 697 g einer 10%igen Natriummethylat-Lösung in Methanol, die eine Konzentration von Wasser und nicht abreagiertem Natriumhydroxid - berechnet als Natriumhydroxid - in Summe von 0,75 Gew.-% aufweist.

### Beispiel 2.2 (nicht erfindungsgemäß):

Als Reaktionskolonne wird die unter Beispiel 2.1 beschriebene Apparatur verwendet. Die Apparatur wird mit 720 g Methanol beschickt, welches zum Sieden erhitzt wird, so dass ca. 460 g Destillat pro h erhalten werden. Am Kopf der Reaktionskolonne wird mit einer Dosierung von 20 g/h eine 50%ige wässrige Natronlauge zudosiert. Die Apparatur wird ohne Rücklauf betrieben. Nach ca. 1 Stunde beobachtet man eine deutliche Feststoffabscheidung auf den obersten 3 Böden der Glockenbodenkolonne.

Nach 6 Stunden wird die Dosierung der Natronlauge am Kopf beendet. Für weitere 30 Minuten wird Methanol am Sumpf der Kolonne zudosiert und mit gleichbleibender Kondensatabnahme weiterdestilliert. Man erhält nach 6,5 Stunden 582 g einer 13%igen Natriummethylat-Lösung in Methanol, die eine Konzentration von Wasser und nicht abreagiertem Natriumhydroxid - berechnet als Natriumhydroxid - in Summe von 0,57 Gew.-% aufweist.

### Beispiel 2.3 (erfindungsgemäß):

Als Reaktionskolonne wird die unter Beispiel 2.1 beschriebene Apparatur verwendet. Die Apparatur wird mit 720 g Methanol beschickt, welches zum Sieden erhitzt wird. Der Rücklaufteiler wird auf ein Rücklaufverhältnis von 0,16 eingestellt, so dass ca. 460 g Destillat pro h erhalten werden. Am Kopf der Reaktionskolonne wird mit einer Dosierung von 20 g/h eine 50%ige wässrige Natronlauge zudosiert. Es wird keine Feststoffausfällung beobachtet.

Nach 6 Stunden wird die Dosierung der Natronlauge am Kopf beendet. Für weitere 30 Minuten wird Methanol am Sumpf der Kolonne zudosiert und mit gleich bleibender Kondensatabnahme weiterdestilliert. Man erhält nach 6,5 Stunden 814 g einer 11 %igen Natriummethylat-Lösung in Methanol, die eine Konzentration von Wasser und nicht abreagiertem Natriumhydroxid - berechnet als Natriumhydroxid - in Summe von 0,57 Gew.-% aufweist.

### Analysenmethode:

Die Bestimmung der Konzentration an Wasser und nicht abreagiertem Alkalimetallhydroxid erfolgt mittels einer modifizierten Karl-Fischer-Titration, wie sie üblicherweise zur Bestimmung von Wassergehalten eingesetzt wird. Hierbei werden Wasser und Alkalimetallhydroxid gemeinsam als Summenparameter detektiert. Die Angabe des entsprechenden Gehaltes erfolgt in Gew.-% - berechnet als Alkalimetallhydroxid.

## Patentansprüche

1. Verfahren zur Herstellung einer alkoholischen Lösung eines Alkalimetallalkoholats aus Alkalimetallhydroxid und Alkohol in einer Reaktionskolonne, wobei der Alkohol und das Alkalimetallhydroxid im Gegenstrom geführt werden,
**dadurch gekennzeichnet,**
**dass** ein Rücklaufverhältnis von mindestens 0,05 an der Reaktionskolonne eingestellt wird und als Alkohole aliphatische Alkohole mit 1 bis 5 Kohlenstoffatomen eingesetzt werden.

2. Verfahren gemäß Anspruch 1,
**dadurch gekennzeichnet,**
**dass** das Wasser im Kondensat und/oder Rücklauf der Reaktionskolonne vor der Rückführung als Rücklauf in die Reaktionskolonne nicht abgetrennt wird.

3. Verfahren gemäß Anspruch 1 oder 2,
**dadurch gekennzeichnet,**
**dass** das Alkalimetallhydroxid in Form einer wässrigen, alkoholischen oder einer wässrigen Lösung, die den eingesetzten Alkohol als weiteres Lösemittel aufweist, eingesetzt wird.

4. Verfahren gemäß zumindest einem der Ansprüche 1 bis 3,
**dadurch gekennzeichnet,**
**dass** die Lösung des Alkalimetallhydroxids am Kopf der Reaktionskolonne zugeführt wird.

5. Verfahren gemäß zumindest einem der Ansprüche 1 bis 4,
**dadurch gekennzeichnet,**
**dass** der Alkohol gasförmig mindestens zwanzig Böden unterhalb der Zulaufstelle der Lösung des Alkalimetallhydroxids zugeführt wird.

6. Verfahren gemäß zumindest einem der Ansprüche 1 bis 5,
**dadurch gekennzeichnet,**
**dass** das Destillat, welches nicht der Reaktionskolonne zurückgeführt wird, in einem weiteren Verfahrensschritt in Wasser und den Alkohol aufgetrennt wird, und dieser Alkohol der Reaktionskolonne zurückgeführt wird.

7. Verfahren gemäß Anspruch 6,
**dadurch gekennzeichnet,**
**dass** der in die Reaktionskolonne zudosierte Alkohol einen durchschnittlichen Wassergehalt von 200 bis 450 ppm (m/m) aufweist.

8. Verfahren gemäß zumindest einem der Ansprüche 1 bis 7,
**dadurch gekennzeichnet,**
**dass** als Alkohol Methanol eingesetzt wird.

9. Verfahren gemäß Anspruch 8,
**dadurch gekennzeichnet,**
**dass** das Destillat der Reaktionskolonne in einer Rektifikationskolonne aufgearbeitet wird.

10. Verfahren gemäß Anspruch 9,
**dadurch gekennzeichnet,**
**dass** die Rektifikationskolonne einen höheren Druck als die Reaktionskolonne aufweist.

11. Verfahren gemäß Anspruch 10,
**dadurch gekennzeichnet,**
**dass** das Destillat, welches nicht der Reaktionskolonne zurückgeführt wird, in einem weiteren Verfahrensschritt in Wasser und Methanol aufgetrennt wird, wobei dieses Destillat über einen Brüdenverdichter in die Rektifikationskolonne geführt wird.

12. Verfahren gemäß Anspruch 9,
**dadurch gekennzeichnet,**
**dass** die Reaktionskolonne einen höheren Druck als die Rektifikationskolonne aufweist.

13. Verfahren gemäß Anspruch 12,
**dadurch gekennzeichnet,**
**dass** das Methanol nach der Abtrennung des Wassers in einer Rektifikationskolonne über einen Brüdenverdichter der Reaktionskolonne wieder zurückgeführt wird.

14. Verfahren gemäß zumindest einem der Ansprüche 9 bis 13,
**dadurch gekennzeichnet,**
**dass** die Zugabe des Frischalkohols in den Kondensator oder in den Kondensatbehälter an der Rektifikationskolonne erfolgt.

## Claims

1. Process for preparing an alcoholic solution of an alkali metal alkoxide from alkali metal hydroxide and alcohol in a reaction column, the alcohol and the alkali metal hydroxide being conducted in countercurrent,
**characterized in that**
a reflux ratio of at least 0.05 is established in the reaction column and the alcohols used are aliphatic alcohols having 1 to 5 carbon atoms.

2. Process according to Claim 1,
**characterized in that**
the water is not removed in the condensate and/or reflux of the reaction column before the recycling as reflux into the reaction column.

3. Process according to Claim 1 or 2,
**characterized in that**
the alkali metal hydroxide is used in the form of an aqueous solution, alcoholic solution or an aqueous solution which comprises the alcohol used as a further solvent.

4. Process according to at least one of Claims 1 to 3,
**characterized in that**
the solution of the alkali metal hydroxide is fed at the top of the reaction column.

5. Process according to at least one of Claims 1 to 4,
**characterized in that**
the alcohol is fed in gaseous form at least twenty trays below the feed point of the solution of the alkali metal hydroxide.

6. Process according to at least one of Claims 1 to 5,
**characterized in that**
the distillate which is not recycled to the reaction column is separated in a further process step into water and the alcohol, and this alcohol is recycled to the reaction column.

7. Process according to Claim 6,
**characterized in that**
the alcohol metered into the reaction column has an average water content of 200 to 450 ppm (m/m).

8. Process according to at least one of Claims 1 to 7,
**characterized in that**
the alcohol used is methanol.

9. Process according to Claim 8,
**characterized in that**
the distillate of the reaction column is worked up in a rectification column.

10. Process according to Claim 9,
**characterized in that**
the rectification column has a higher pressure than the reaction column.

11. Process according to Claim 10,
**characterized in that**
the distillate which is not recycled to the reaction column is separated in a further process step into water and methanol, and this distillate is conducted into the rectification column via a vapour compressor.

12. Process according to Claim 9,
**characterized in that**
the reaction column has a higher pressure than the rectification column.

13. Process according to Claim 12,
**characterized in that**
the methanol, after the removal of the water in a rectification column, is recycled back to the reaction column via a vapour compressor.

14. Process according to at least one of Claims 9 to 13,
**characterized in that**
the fresh alcohol is added to the condenser or to the condensate vessel on the rectification column.

## Revendications

1. Procédé pour la production d'une solution alcoolique d'un alcoolate de métal alcalin à partir d'hydroxyde de métal alcalin et d'alcool dans une colonne de réaction, l'alcool et l'hydroxyde de métal alcalin étant envoyés à contre-courant, **caractérisé en ce qu'**on règle dans la colonne de réaction un taux de reflux d' au moins 0,05 et on utilise comme alcools des alcools aliphatiques ayant de 1 à 5 atomes de carbone.

2. Procédé selon la revendication 1, **caractérisé en ce que** l'eau dans le condensat et/ou le reflux de la colonne de réaction avant le recyclage en tant que reflux dans la colonne de réaction n'est pas séparée.

3. Procédé selon la revendication 1 ou 2, **caractérisé en ce que** l'hydroxyde de métal alcalin est utilisé sous forme aqueuse, alcoolique ou d'une solution aqueuse qui comporte comme autre solvant l'alcool introduit.

4. Procédé selon au moins l'une des revendications 1 à 3, **caractérisé en ce que** la solution de l'hydroxyde de métal alcalin est introduite à la tête de la colonne de réaction.

5. Procédé selon au moins l'une des revendications 1 à 4, **caractérisé en ce que** l'alcool à l'état gazeux est introduit à au moins vingt plateaux au-dessous du point d'introduction de la solution de l'hydroxyde de métal alcalin.

6. Procédé selon au moins l'une des revendications 1 à 5, **caractérisé en ce que** le distillat qui n'est pas renvoyé à la colonne de réaction est séparé en eau et l'alcool dans une autre étape du processus, et cet alcool est renvoyé à la colonne de réaction.

7. Procédé selon la revendication 6, **caractérisé en ce que** l'alcool introduit par addition dosée dans la colonne de réaction présente une teneur moyenne en eau de 200 à 450 ppm (m/m).

8. Procédé selon au moins l'une des revendications 1 à 7, **caractérisé en ce que** comme alcool on utilise le méthanol.

9. Procédé selon la revendication 8, **caractérisé en ce que** le distillat de la colonne de réaction est soumis à un traitement final dans une colonne de rectification.

10. Procédé selon la revendication 9, **caractérisé en ce que** la colonne de rectification présente une pression supérieure à celle de la colonne de réaction.

11. Procédé selon la revendication 10, **caractérisé en ce que** le distillat qui n'est pas renvoyé à la colonne de réaction est séparé en eau et méthanol dans une autre étape du processus, ce distillat étant envoyé à la colonne de rectification en passant par un compresseur de vapeur.

12. Procédé selon la revendication 9, **caractérisé en ce que** la colonne de réaction présente une pression supérieure à celle de la colonne de rectification.

13. Procédé selon la revendication 12, **caractérisé en ce qu'**après la séparation de l'eau dans une colonne de rectification le méthanol est de nouveau renvoyé à la colonne de réaction en passant par un compresseur de vapeur.

14. Procédé selon au moins l'une des revendications 9 à 13, **caractérisé en ce que** l'addition de l'alcool neuf dans le condenseur ou dans le récipient de condensat s'effectue au niveau de la colonne de rectification.
